(19) Europäisches Patentamt
European Patent Office
Office européen des brevets

(11) Publication number: **0 307 476**
**A1**

(12) # EUROPEAN PATENT APPLICATION

published in accordance with Art. 158(3) EPC

(21) Application number: **88900121.0**

(22) Date of filing: **19.12.87**

Data of the international application taken as a basis:

(86) International application number:
**PCT/JP87/00999**

(87) International publication number:
**WO88/04670 (30.06.88 88/14)**

(51) Int. Cl.³: **C 07 K 15/12**
C 07 K 15/04, A 61 K 39/395
C 12 P 21/00, G 01 N 33/577
G 01 N 33/72

(30) Priority: **20.12.86 JP 304698/86**
**27.04.87 JP 105496/87**

(43) Date of publication of application:
**22.03.89 Bulletin 89/12**

(84) Designated Contracting States:
**CH DE FR GB IT LI SE**

(71) Applicant: **KUKITA, Takeshi**
**1-335-501, Kitaochiai 5-chome**
**Suma-ku Kobe-shi Hyogo 654-01(JP)**

(72) Inventor: **NAKAJIMA, Hiroshi**
**30-14, Takaido Higashi 3-chome**
**Suginami-ku Tokyo 168(JP)**

(74) Representative: **LOUIS, PÖHLAU, LOHRENTZ & SEGETH**
**Ferdinand-Maria-Strasse 12**
**D-8130 Starnberg(DE)**

(54) **BILIRUBIN ANTIGEN, MONOCLONAL ANTIBODY THEREFOR, PROCESS FOR THEIR PREPARATION, AND THEIR USE.**

(57) A novel bilirubin antigen capable of functioning as an antigen which does not undergo decomposition *in vivo*. This antigen has a structure wherein a protein is bound to the methylene group of the outer vinyl group present in the outermost ring of bilirubin through a spacer or to at least one carboxyl group. A monoclonal antibody capable of recognizing the bilirubin antigen can be obtained from hybridoma cells prepared by cell fusion between antibody-yielding cells obtained by immunizing with the bilirubin antigen and tumor cells capable of division and growth in a culture medium. This monoclonal antibody can recognize bilirubins present *in vivo* without decomposing their structures, and hence it enables direct qualitative and analyses of bilirubins.

EP 0 307 476 A1

# SPECIFICATION

## BILIRUBIN ANTIGEN, MONOCLONAL ANTIBODY THEREFOR, PROCESS FOR THEIR PRODUCTION, AND THEIR USE

### Technical Field

The present invention relates to a bilirubin antigen, a monoclonal antibody therefor, processes for their production, and their use. More particularly, it relates to a monoclonal antibody having a specificity to bilirubin, an antigen to the monoclonal antibody, processes for production thereof, and their use.

### Background Art

The heme segment of the heme proteins such as hemoglobin, which has lost its functions in the living body, is converted to biliverdin by decomposing its porphyrin ring and then reduced to bilirubin in order to recover iron components therein.

Bilirubin present in vivo may be classified roughly into two: one being a free bilirubin which is soluble in oil (of the type being indirectly reactive in the diazo reaction) and the other being salts and esters of bilirubin which are soluble in water (of the type being directly reactive in the diazo reaction) (see Yamaoka et al.: Proc. Japan Acad., Vol. 27, 715-721, 1951). Furthermore, the free bilirubin includes isomers, and it is confirmed that IX $\alpha$ isomer accounts for approximately 97% of bilirubin in the adult bile juice and small amounts of IX $\beta$ as well as IX$\delta$ isomers are contained therein. A variety of derivatives in the bilirubin esters are reported which may include monoesters such as monoglucuronate conjugate, diesters such as diglucuronate conjugates, diglucose conjugates, and dixylose conjugates, and so on. As bilirubin conjugates in the ester form are known, for example, di-conjugates with glucuronic acid, glucose, xylose and so on, and mono-conjugates with glucuronic acid and so on.

There is yet no commercially available method for directly

quantitating these bilirubins and derivatives. Amounts of the bilirubins are to be indirectly determined by decomposing them by means of the diazo reaction and measuring the resulting diazo compounds. It is noted, however, that the free bilirubin and conjugates are so unstable that they may be decomposed easily during the diazo reaction and during operation of extraction or isolation. Accordingly, this diazo method suffers from the disadvantage that the free bilirubin and the bilirubin conjugates cannot be analyzed in a quantitative manner. Furthermore, it presents the disadvantage that values obtainable from quantitative analysis may be caused to vary to a great extent on account of substances forming azo pigments other than the bilirubins and of other substances present in the living body, such as carotines providing yellow color. The conventional bilirubin analysis method can measure only the bilirubins of the directly reactive type and a total bilirubin amount. This analysis is to measure the bilirubins after chemically decomposing the original bilirubin so that it suffers from the critical disadvantage that neither the cause of jaundice nor disease conditions can be determined because the original structures of the bilirubins prior to chemical decomposition cannot be identified. Accordingly, strong demands have been made to provide an analysis method that can directly quantitate the free blirubin and the bilirubin conjugates or derivatives without decomposition of their structures in vivo.

One of such a direct bilirubin analysis method is proposed to use a high performance liquid chromatography (HPLC) (see Yamaguchi et al.: Proc. Japan Acad., 55B, 89-93, 1979). This HPLC method permits a quantitation of the free bilirubin and the bilirubin conjugates with high sensitivity and without decomposition of their original structures in vivo. This method, however, suffers from the disadvantages that it employs an expensive device, it requires as long as about one hour for analyzing one sample, and it requires an operator with high skill as well as it does not necessarily provide

the guarantee that separated fractions consist of a uniform substance.

Furthermore, the inventor has completed another direct quantitative analysis method that permits a ready quantitation of the free bilirubin and bilirubin conjugates within a short period of time without decomposition of their original structures, while their in vivo structures are retained. This method involves the use of a monoclonal antibody that was formed using the bilirubins as antigens (Japanese Patent Publication (laid-open) No. 5025/1986). This method, however, suffers from the disadvantage that the monoclonal antibody used therefor cannot recognize the in vivo steric structures of the bilirubins. Accordingly, in order to precisely quantitate the bilirubins present in vivo and accurately determine the cause and disease conditions of jaundice, a strong demand has still been made to provide a method using a monoclonal antibody capable of recognizing even the steric structures of the bilirubins in vivo.

Therefore, the present invention has the object to provide a novel bilirubin antigen that can function as an antigen in such a state as reflecting the structure of the bilirubin present in vivo.

The present invention has another object to provide a monoclonal antibody that can recognize the bilirubin antigen with high accuracy and high sensitivity.

The present invention has a further object to provide processes for preparing the bilirubin antigen and the antibody thereto.

The present invention has a still further object to provide the use of the antibody for recognizing and detecting the bilirubin antigen.

It is now to be noted that the terms "bilirubin" or "bilirubins" used in this specification are meant to include a bilirubin conjugate, bilirubin C, various bilirubin isomers and bilirubin metabolites as well as free bilirubin and biliverdin unless otherwise specified or unless technically available.

Disclosure of Invention

The novel bilirubin antigen according to the present invention is

one capable of being recognized and detected as an antigen by the monoclonal antibody that is in such a state that the bilirubin in vivo is measured as it is without being transformed or decomposed. Such bilirubin antigens may be obtained, for example, by connecting the bilirubin to a protein segment without or with interposition of a spacer therebetween. More specifically, the bilirubin antigen with the bilirubin connected to the protein segment through the spacer, on the one hand, is in such a form that the carbon atom in the most external vinyl group in the pyrroline ring of the bilirubin is connected to the protein segment through the spacer. The bilirubin antigen with the bilirubin connected to the protein segment without the aid of any spacer, on the other hand, is in such a form that at least one of two carboxyl groups of the bilirubin is connected directly to the protein segment.

The bilirubin as one of the starting material for forming the bilirubin antigens according to the present invention may include, for example, bilirubin conjugates, bilirubin C, various bilirubin isomers and bilirubin metabolites as well as free bilirubin and biliverdin.

The spacer to be used for the preparation of the bilirubin antigen obtainable by connecting the bilirubin to the protein segment therethrough may be any substance capable of connecting the most external vinyl group of the bilirubin to the protein segment and retaining the function as an antigen without transforming the structure of the bilirubin.

More specifically, the bilirubin antigen obtainable by connection of the bilirubin residue to the protein segment through the spacer may be represented by general formula (A):

bilirubin residue - $CH(CH_3)$ - SP - N - PT

in which "bilirubin residue" stands for a residue of the bilirubin from which the most external vinyl group is excluded, the bilirubin including free bilirubin, biliverdin, a bilirubin conjugate, bilirubin

C. a bilirubin isomer or a bilirubin metabolite; SP stands for a spacer residue, and PT stands for a protein segment.

The spacer may include, for example, thiols and alcohols such as an aldehyde thiol, an aldehyde alcohol, a substituted aldehyde thiol and a substituted aldehyde alcohol, and amino group-containing thiols.

More specifically, the thiols and the alcohols may be represented by general formula (I) as follows:

$$H - X - R_1 - CHO$$

(in which X stands for a sulfur atom or an oxygen atom; and $R_1$ stands for a saturated or unsaturated aliphatic, aromatic, or araliphatic residue.)

It is to be noted herein that the aliphatic residue as it stands and in the araliphatic residue may include a straight or branched, divalent hydrocarbon residue having from one to six carbon atoms and that the aliphatic and aromatic segments may have an appropriate substituent or substituents.

The thiols may include, for example, a saturated aliphatic aldehyde thiol, an unsaturated aliphatic aldehyde thiol, an aromatic aldehyde thiol, and an araliphatic aldehyde thiol. Representatives of the saturated aliphatic aldehyde thiols may be acetoaldehyde thiol, propionaldehyde thiol, butylaldehyde thiol, isobutylaldehyde thiol, valeraldehyde thiol, laurylaldehyde thiol and so on. Representatives of the unsaturated aliphatic aldehyde thiols may be mercaptoacrolein and crotonaldehydethiol and so on. Representatives of the aromatic aldehyde thiols may include mercaptobenzaldehyde, mercaptotolualdehyde and so on. Representatives of the araliphatic aldehyde thiols may be mercaptobenzyl aldehyde, mercaptophenethyl aldehyde, mercaptoethylphenyl benzaldehyde, mercaptomethylphenethyl aldehyde and so on.

The aldehyde alcohols may include, for example, one in which an hydroxyl group is connected to the aliphatic, aromatic or araliphatic segment of the saturated or unsaturated aliphatic, aromatic or araliphatic aldehyde. More specifically, the aldehyde alcohols are

formed by substituting the hydroxyl group for the thiol group in the saturated or unsaturated aliphatic, aromatic or araliphatic aldehyde thiols, and representatives of the aldehyde alcohols may include, for example, glycol aldehyde, ethyleneglycol aldehyde, propyleneglycol aldehyde, butaneglycol aldehyde, pinacolaldehyde, formylbenzyl alcohol, formylphenethyl alcohol, hydroxymethylphenyl aldehyde, hydroxyethylphenyl aldehyde and so on.

These aldehyde thiols and aldehyde alcohols may be provided with a variety of substituents such as a hydroxyl group, a halogen atom, nitro group, a carboxyl group or the like.

The aldehyde group in the thiols or alcohols as represented by the general formula (I) above may be converted into a Schiff's base by reaction with an amino group of the protein segment, thus forming a bilirubin antigen. In this case, the bilirubin may be reacted first with the thiol or the alcohol and then with the protein segment or the thiol or the alcohol may be reacted first with the protein segment and then the resulting product is then reacted with the bilirubin.

The protein residue as represented by "PT" constituting the bilirubin antigen according to the present invention may be any protein residue or segment capable of forming the bilirubin antigen, such as albumin, hemocyanine, thyroglobulin, ovalbumin and so on.

Another type of the bilirubin antigens according to the present invention may be a bilirubin antigen as represented by general formula (IVa):

bilirubin residue - $CH(CH_3)$ - X - $Y_1$ - N=CH - $Y_2$ -CH=N - PT

(in which $Y_1$ stands for an unsubstituted or substituted lower alkylene group having from one to six carbon atoms; $Y_2$ stands for a dialdehyde residue; and "bilirubin residue", X and PT have the same meaning as above).

The bilirubin antigen of formula (IVa) may be obtainable by converting a bilirubin compound as represented by general formula (II):

bilirubin residue - $CH(CH_3)$ - X - $Y_1$ - $NH_2$ (in which "bilirubin residue", X and $Y_1$ have the same meanings as above):

into a bilirubin-aldehyde compound as represented by general formula (III):

bilirubin residue - $CH(CH_3)$ - X - $Y_1$ - N=CH - $Y_2$ - CHO

(in which "bilirubin residue", X, $Y_1$ and $Y_2$ have the same meanings as above),

and then by reacting the resulting bilirubin-aldehyde compound with the protein residue or segment.

Furthermore, there may be obtained another bilirubin antigen represented by general formula (IVb):

bilirubin residue - $CH(CH_3)$ - X - $Y_1$ - $NH-CH_2$ - $Y_2$ $-CH_2-NH$ - PT

(in which "bilirubin residue", X, $Y_1$ and $Y_2$ have the same meanings as above)

by reduction of the bilirubin antigen of the general formula (IVa).

The lower alkylene group represented by $Y_1$ in the general formula (II) may be a straight or branched, divalent saturated aliphatic hydrocarbon residue having from one to six carbon atoms and may be provided with a substituent such as a hydroxyl group, a carboxyl group or the like. The lower alkylene group may include, for example, methylene, ethylene, propylene, methylpropylene, dimethylpropylene, hexylene, hydroxylethylene, carboxylethylene and so on.

The bilirubin compound as represented by the general formula (II) may be prepared by connecting the bilirubin to am amino group-containing thiol or alcohol. The amino group-containing thiol or alcohol may be a compound having both an amino group and a sulfhydryl group (SH) which may include, for example, cysteine, cysteamine and so on. The reaction of the bilirubin with the amino group-containing thiol or alcohol may be preferably carried out in an acidic condition using a sulfonic acid such as toluenesulfonic acid. As the bilirubin to be used as a starting material is unstable, the

reaction may be preferably conducted under mild conditions and at or below ambient temperatures.

The bilirubin-aldehyde compound as represented by the general formula (III) may be prepared by reacting the bilirubin compound of the general formula (II) with a dialdehyde compound as represented by general formula:

$$OHC-(CH_2)_n-CHO$$

(in which n is an integer).

Such a dialdehyde compound may include, for example, malondialdehyde, succindialdehyde, glutardialdehyde, adipindialdehyde and so on. This reaction permits the amino group of the bilirubin compound of the general formula (II) to form a Schiff's base by connection to one of the aldehyde groups in the dialdehyde compound as represented above.

The aldehyde group of the bilirubin-aldehyde compound as represented by the general formula (III) may form a Schiff's base by reaction with an amino group on the protein segment, thus forming the bilirubin antigen as represented by the general formula (IVa) above.

The bilirubin antigen as represented by the general formula (IVa) above may be reduced in conventional manner to the bilirubin antigen as represented by the general formula (IVb) above.

A further type of the bilirubin antigens may include one which is obtainable by reaction of the bilirubin compound as represented by the general formula (II) above with a bifunctional cross-linkable compound to give a compound as represented by general formula (V):

bilirubin residue $- CH(CH_3) - X - Y_1 - N = Y_3$

(in which $Y_3$ stands for a residue of the bifunctional cross-linkable compound and "bilirubin residue", X and $Y_1$ have the same meanings as above)

and then converting the compound of the general formula (V) into a Schiff's base as represented by general formula (VIa):

bilirubin residue $- CH(CH_3) - X - Y_1 - N = Y_3' = N - PT$

(in which $Y_3'$ stands for a residue of the bifunctional

cross-linkable compound and "bilirubin residue", X and $Y_1$ have the same meanings as above)

by reaction with the protein residue of segment; or one which is obtainable by reduction of the Schiff's base of the general formula (VIa) to a bilirubin antigen as represented by general formula (VIb):

bilirubin residue - $CH(CH_3)$ - X - $Y_1$ - NH - $Y_3''$ - NH - PT

(in which $Y_3''$ stands for a reduced form of $Y_3'$ and "bilirubin residue", X and $Y_1$ have the same meanings as above).

The bilirubin compound as represented by the general formula (II) may be reacted separately with the bifunctional cross-linkable compound and with the protein segment or may be reacted simultaneously with the other two, thereby leading to the bilirubin antigen as represented by the general formula (VIa) which in turn is reduced in the same manner as above to the bilirubin antigen as represented by the general formula (VIb) above.

The compound as represented by the general formula (V) may be formed by reaction of the bilirubin compound of the general formula (II) with the bifunctional cross-linkable compound at pH ranges from approximately 7 to 11. The bifunctional cross-linkable compound may include, for example, a disuccinimidyl compound such as disuccinimidyl tartrate, ethylene glycol bis(disuccinimidyl succinate) and so on, a bissulfosuccinimidyl compound such as bis(sulfosuccinimidyl) suberate and so on, and a fluoro-dinitrobenzene compound such as 1,5-difluoro-dinitrobenzene and so on.

The bilirubin antigens obtainable in the form of Schiff's bases may be reduced to the corresponding reduced form. A reducing agent to be used for the reduction may be any agent which is conventionally used for this purpose and which permits reduction of the Schiff's base without exerting any undesirable influence upon the other sites. Such a reducing agent may include, for example, a metal borohydride

including an alkali metal borohydride such as sodium borohydride, potassium borohydride, sodium cyanoborohydride, and so on.

The bilirubin antigens obtainable by connection to the protein segment without the aid of the spacer may include, for example, one in which at least one of the two carboxyl groups on the bilirubin is bound to the protein segment. It is accordingly to be understood that the bilirubins to be used in this case should be one other than the one in which the two carboxyl groups on the bilirubin are both conjugated with glucuronic acids or the like.

The bilirubin antigens of the type may be obtained by binding the protein segment to the carboxyl group of the bilirubin in conventional manner using an acylating agent, a condensing agent or the like. The acylating agent to be used may include, for example, an inorganic or organic base salt, an acid anhydride, an acid halide or the like. The inorganic base salt may include a salt with an alkali metal such as sodium, potassium or the like or an alkaline earth metal such as calcium or the like. As the organic base salt may be exemplified a salt with a tertiary amine such as trimethylamine, triethylamine, dibenzylmethyl amine or the like. As the acid halide may be exemplified an acid chloride, an acid bromide or the like. The acid anhydride may include isobutyl chloroformate or the like. Representatives of the condensing agents may include, for example, a carbodiimide compound such as N,N'-dicyclohexyl carbodiimide, N-cyclohexyl-N'-(4-diethylaminocyclohexyl)carbodiimide, N,N'-diethylcarbodiimide, N,N'-diisopropylcarbodiimide and N-ethyl-N'-(3-dimethyl- aminopropyl)carbodiimide, a ketenimine compound such as N,N'-carbonyl- bis(2-methylimidazole), pentamethyleneketene-N-cyclohexylimide and diphenylketene-N-cyclohexylimide, an N-hydroxybenzotriazole compound such as 1-(4-chlorobenzenesulfonyloxy)-6-chloro-1H-benzotriazole, and so on. This reaction may be carried out in the presence of a conventional solvent such as water, acetone, dioxane, acetonitrile,

chloroform, benzene, methylene chloride, tetrahydrofuran, ethyl acetate or the like. Reaction temperatures are not limited to particular ones and the reaction may be preferably conducted under mild conditions such as, for example, under cooling.

The monoclonal antibody according to the present invention is meant herein to refer to a monoclonal antibody that can accurately recognize the corresponding bilirubin without transformation or decomposition of its structure and state present in vivo. Accordingly, it should be understood herein that the monoclonal antibody according to the present invention is not restricted to the one capable of recognizing the bilirubin antigen as have been enumerated hereinabove as representative examples.

The monoclonal antibody according to the present invention may be produced from a hybridoma cell obtainable by fusing an antibody-producing cell with a tumor cell, the antibody-producing cell being immuned with an antigen bound with the bilirubin and the tumor cell being capable of promulgating by division in the culture system. The antigen to be used for the present invention may be one obtainable by binding the bilirubin to the protein segment. This antigen will be referred to simply as the bilirubin antigen unless otherwise specified. The bilirubin may include free bilirubin, biliverdin, a bilirubin conjugate including a mono-conjugate with glucuronic acid, glucose, xylose or the like, or a bilirubin derivative or isomer such as bilirubin C, in which at least one of the two carboxyl group on the bilirubin is in a free state. The protein for forming the bilirubin antigen by connection to the bilirubin may include, for example, a protein segment derived from animals, such as haemocyanine, albumin, ovalbumin, thyroglobulin or the like.

As the bilirubin to be used for the antigen is an isomer or derivative of the bilirubin, animals such as mice may be immuned with a bilirubin antigen as a sole substance or as a mixture with other bilirubin antigen or antigens.

In events where the mixture of the bilirubin antigens is used, antibodies produced thereby may be screened to separate hybridoma cells containing antibodies having specificities to the corresponding bilirubins used for immunization.

In accordance with the present invention, methods for immuning animals such as mice, rats or the like with the bilirubin may be conventional ones. An amount of the bilirubin antigen, and the number and timings of immunization may vary with the immunoresponsive ability of the animals and degrees of purification of the antigen. In the event where the amount of the bilirubin antigen is too small, immunity is rendered so insufficient that an additional application of the antigen is required. In usual cases, however, it is preferred to carry out an additional immunization in order to ensure a sufficient degree of immunity. The immunization may be generally carried out using an adjuvant such as Freund's adjuvant or alum.

The antibody producing cells may be prepared from organs of the animals in several days after final immunization. The preparation may be preferably made in three or four days after the final immunization. The antibody producing cells so prepared are preferably fused with the tumor cells as soon as possible. A variety of procedures may be preferably adopted to enhance a fusion efficiency - for example, by breaking erythrocytes in the cells prior to cell fusion.

Among the tumor cells to be used for fusion, myeloma cell strains such as, for example, P3·X63·Ag(X63), P3·X63·Ag8(U1) or P3·NS-1/1·Ag4·(NS-1) are most preferable. The myeloma cell strains to be used are usually of the origin of a BALB/c mouse, but they may be ones originated from a mouse other than BALB/c strain or obtainable from rat cells. They permits a high fusion efficiency for forming hybridoma cells. It is to be noted herein that it is preferred to correspond the kind of the animal originating the myeloma cell strains with the kind of the animal originating the antibody-producing

cells. For cell fusion, it is further preferred to use cells in the logarithmic phase.

A culture medium to be used for promulgation of the hybridoma cells may be usually NS-1 culture medium. As a basic medium for this culture medium may be employed RPMI, and L-glutamine or pyruvic acid may be added. To the NS-1 culture medium may be added bovine fetus serum (FCS).

In accordance with the present invention, the cell fusion may be carried out in conventional manner using a cell fusion reagent such as polyethylene glycol or by means of the electrical cell fusion method. The polyethylene glycol to be used for this purpose may be one having a variety of average melecular weights generally including 1,000, 1,500, 2,000, 4,000 or 6,000. Concentrations of polyethylene glycol solutions may usually range from approximately 30% to 50%. An order of adding the polyethylene glycol solution is not restricted at all to any particular one, and the polyethylene glycol solution may be added to a mixture of the antibody-producing cells with the myeloma cells follwed by the stirring or mixing of the mixture or a mixture of the antibody producing cells with the myelome cells may be admixed with the polyethylene glycol solution and the resulting mixture may be stirred or centrifuged at low speeds, thus leading to cell fusion. Furthermore, a ratio of the antibody-producing cells to the tumor cells is not limited to a particular one and may preferably range from a 1-to-1 ratio to a 20-to-1 ratio.

As unfused cells are still contaminated with the hybridoma cells obtained by the above cell fusion, the unfused cells may be removed by subjecting the resulting cells to incubation in a culture medium such as hypoxanthine-aminopterin-thymidine medium (HAT madium), thus leading to a selection of the hybridome cells only.

The antibody activity of the hybridoma cells contained in a supernatant is then measured for cloning the cells having the objective antibody activity. The cloning may be effected in

conventional manner. A supernatant thus obtained is then measured for its antibody activity and the cells having positive antibody activity are further incubated. The clonings may be effected preferably twice or more.

The clones having the objective antibody activity are then incubated on a largle scale and injected intraperitoneally into animals such as mice having the histocompatible antigen. The monoclonal antibody according to the present invention may be prepared from the abdominal dropsy of the animals injected with the clones. It is to be noted herein that, in addition to the use of the abdominal dropsy, the monoclonal antibody may also be produced using a culture medium containing no serum or using animal cells and culturing them in vitro. The monoclonal antibody thus obtained may be stored by lyophilization so that it can provide a uniform quality. Accordingly, the monoclonal antibody according to the present invention is extremely useful for practical usage.

Modes for Carrying Out the Invention

Example 1: Preparation of Bilirubin Antigen:

Bovine serum albumin (10 mg) was dissolved in 2.0 ml of dioxane leading to a bovine serum albumin solution (BSA).

Separately, 10 mg of bilirubin was dissolved in 1.0 ml of dimethylsulfoxide, and the bilirubin was activated by the addition of 3.0 μl of tri-n-butylamine and 2.0 μl of isobutyl chloroformate. To this mixture was added 50μl of 0.1N sodium hydroxide, and the bovine serum albumin solution prepared above was then added dropwise to the mixture. To this mixture was added 1N hydrochloric acid or sodium hydroxide to adjust a pH of the resulting mixture to about 9.5, and the mixture is then stirred at 4℃ for 12 hours in order to complete the coupling. The reaction mixture thus obtained was then subjected to gel filtration using Cephadex G-25 thus yielding bilirubin-bound BSA (bilirubin 8 moles/protein 1 mole).

Example 2: Preparation of Bilirubin Antigen:

The procedures of Example 1 were followed with the exception that human serum albumin (HSA) fraction V was employed in place of bovine serum albumin (BSA) leading to the preparation of bilirubin-bound HSA (bilirubin 11 moles/protein 1 mole).

Example 3: Preparation of Bilirubin Antigen:

The procedures of Example 1 were followed with the exception that chicken egg albumin (ovalbumin) was employed in place of bovine serum albumin (BSA) leading to the preparation of bilirubin-bound ovalbumin (bilirubin 6 moles/protein 1 mole).

Example 4: Preparation of Bilirubin Antigen:

The procedures of Example 1 were followed with the exception that bovine thyroglobulin (BTG) was employed in place of bovine serum albumin (BSA) leading to the preparation of bilirubin-bound BTG (bilirubin 22 moles/protein 1 mole).

Example 5: Preparation of Bilirubin-Antigen:

Unconjugated bilirubin (UCB) IX$\alpha$ (10 mg) was dissolved in 1.0 ml of dimethyl sulfoxide, and after making sure no formation of any crystal material, 3.0 $\mu$l of tri-n-butylamine and 2.0 $\mu$l of isobutyl chloroformate were added to the resulting solution.

Separately from the above solution, 10 mg of bovine serum albumin was dissolved in 0.024M NaOH, and 2.0 ml of dioxane was added to the soltuion. The mixture was then centrifuged at 10,000G for 5 minutes and undissolved material was removed.

To the above bilirubin soltution was added 50 $\mu$l of NaOH, and the albumin solution prepared separately was added dropwise to the resulting mixture. The mixture was then adjusted to pH 9.5 with NaOH or HCl and stirred overnight at 4 ℃.

The resulting product was purifed by means of gel filtration using Cephadex G-25.

As a result of observation on unbound bilirubin by means of absorption at 450 nm and albumin by means of the protein assay, it

was found that the bound product was a bilirubin antigen in which 8 moles of bilirubin were bound per mole of albumin.

Example 6: Preparation of Bilirubin Antigen:

The procedures of Example 5 were followed in substantially the same manner with the exception that human serum albumin was employed in place of bovine serum albumin, leading to the preparation of a bilirubin antigen in which 8 moles of bilirubin were bound per mole of the albumin.

Example 7: Preparation of Bilirubin Antigen:

Biliverdin was dissolved in methanol and the solution was subjected to thin layer chromatography on a silica gel plate using a developing solution of a chloroform/methanol (80/20 v/v) solution. The thin layer chromatography gave two bands, and the main band was treated as a purified biliverdin in the manner as will be described below. It is further to be noted that this main band has been confirmed by means of high performance liquid chromatography (HPLC) to be an $\alpha$ type biliverdin.

The main band biliverdin was dissolved in a minimum amount of dimethyl formamide, and 500 $\mu$l of this solution was then mixed with 1.7 $\mu$l of isobutyl chloroformate and 3.1 $\mu$l of tri-n-butyl amine. After stirring, the mixture was allowed to stand at room temperature for 20 minutes.

Separately from this bilirubin solution, 30 mg of limulus hemocyanine (KLH) was dissolved in 3.2 ml of distilled water and 0.1 ml of 1N sodium hydroxide, and the solution was centrifuged at 10,000G for removal of undissolveld material.

To the KLH solution was added dropwise the biliverdine solution, and the mixture was adjusted to pH9.1 with hydrochloric acid or sodium hydroxide. The mixture was then allowed to stand overnight at 4 ℃.

The gel filtration of the resulting mixture separated the protein-bound biliverdin and the unbound biliverdin, and the solution

obtained by centrifugation was subjected to column chromatography on Cephadex G-25 using an eluting solution (PBS), thus leading to purification of a bilirubin antigen in which the biliverdin was bound to KLH.

Example 8: Preparation of Bilirubin Antigen:

Bilirubin (0.22 mg) was dissolved in an minimum amount of 1,4-dioxane while being gradually refluxed, and the resulting solution was transferred to a cool chamber in which it was cooled to 8℃ while care is taken so as to form no crystal material. To this solution were added 0.22 mmol of isobutyl chloroformate and 0.22 mmol of tri-n-butylamine. After stirring. the mixture was allowed to stand at 8℃ for 20 minutes. Separately from the bilirubin solution, 4μmol of limulus hemocyanine (KLH) was dissolved in 8 ml of 1,4-dioxane. To this KLH solution was dropwise added the bilirubin solution prepared above. The mixture was adjusted to pH 8.7 to 9.2 with hydrochloric acid or sodium hydroxide and subjected to reaction for 1 hour at 4℃. The solution was further adjusted to the pH above and subjected to reaction at 4 ℃ for 2 hours while being stirred, thereby yielding a bilirubin antigen in which the bilirubin was bound to the limulus hemocyanine.

Example 9: Preparation of Bilirubin Antigen:

Bilirubin was dissolved in a chloroform solution (1 mg/ml) containing 5% p-hydroxybenzaldehyde, and several pieces of crystals of p-toluenesulfonic acid were added to the solution. The mixture was then allowed to stand in a dark place for about 8 hours, then yielding a bilirubin derivative in which formylphenoxy group was bound to the exo-vinyl group of the bilirubin.

The bilirubin derivative thus obtained was reacted with bovine serum albumin in the presence of 0.33M dipotassium hydrogen phosphate adjusted to pH 9 with potassium hydroxide, thereby forming a Schiff's base in which the bilirubin derivative was bound to the albumin.

The resulting Schiff's base was further reduced with sodium

borohydride while being stirred at room temperature to therebty yield the corresponding reduced form.

Example 10: Preparation of Bilirubin Antigen:

The procedures of Example 9 were followed in substantially the same manner with the exception that p-mercaptobenzaldehyde was employed in place of p-hydroxybenzaldehyde, thereby leading to the formation of a bilirubin antigen in which the bilirubin was bound to the albumin through the benzaldehyde.

Example 11: Preparation of Bilirubin Antigen:

The procedures of Example 9 were followed in substantially the same manner with the exception that cysteine was employed in place of p-hydroxybenzaldehyde. This example gave a bilirubin derivative in which the thiol group of the cysteine was bound to the exo-vinyl group of the bilirubin.

The bilirubin derivative (5 mg) was dissoved in 1 ml of 0.1M phosphate buffer (pH 6.8). To this solution was added dropwise 12 mg of bovine serum albumin, and the reaction mixture was allowed to stand at room temperature for 2 hours. The mixture was further dialyzed overnight at 4 ℃ twice using a 5 liter portion of physiological saline to which the phosphate buffer had been added. The formed precipitate was removed by centrifugation at 20,000G and 4 ℃ for 30 minutes, thereby leading to the formation of an albumin-bound bilirubin derivative solution. This solution was stored at -80℃ before being used.

Example 12: Preparation of Bilirubin Antibody:

The bilirubin antigen produced in Example 5 above was emulsified with the same amount of Freund's complete adjuvant, and BALB/c mice were immuned with 100 μl (60 μg) of the emulsified solution by intraperitoneal injection. The antigen was additionally injected at the daily rate of 200 μl, 400μl, 400μl and 400 μl from four days prior to cell fusion.

The spleen cells of the mice immuned above were then admixed with

mouse myeloma cells (P3-X63-Ag8-U1) at the ratio of 10 to 1 (spleen cells to myeloma cells) and the mixture was subjected to cell fusion at 37 ℃ for 8 minutes using 45% polyethylene glycol 6000. The cells thus treated was suspended in a hypoxanthine-thymidine (HT) medium and poured into tissue culture plate wells followed by incubation at 37℃ for 24 hours in an incubator with 5% $CO_2$. After incubation, the HT medium was changed to a HAT medium for selection of hybridoma cells. After incubation for 2 to 3 weeks, occurrence of colonies was visually observed. A supernatant of the colonies was removed, and the production of the desired monoclonal antibody was checked using the ELISA method.

The colonies which had been found positive in the monoclonal antibody production by the above method were further incubated with mice spleen cells.

(Screening)

The UCB-HSA bilirubin solution (1 $\mu$l in 100$\mu$l of phosphate buffer-containing physiological saline (PBS)) was allowed to stand overnight at 4℃ in polystyrene plate wells and the wells were washed three times with PBS to remove the unbound antigen. After pouring 1% gelatin (PBS) solution into the wells and allowing them to stand at room temperature for 1 hour, they were washed three times with three portions of a PBS solution containing 0.05% Tween 20 for removal of the unbound protein. The supernatant containing the antibody was diluted by ten times with 1% HSA-PBS and 10 $\mu$l of an aliquot of the antibody supernatant was added to the wells. The wells were incubated at 37 ℃ for 30 minutes and then washed three times with PBS containing 0.05% Tween 20. To them was added rabbit anti-mouse IgG(Fab')$_2$-urease- conjugate (diluted in advance to 100 times with 0.25%BSA-PBS). After the wells were further incubated at 37 ℃ for 30 minutes and washed three times with PBS containing 0.05% Tween 20, 100 $\mu$l of a substrate solution was added to each of the wells andthe wells were allowed to stand at room temperature for 30 minutes. They were

then measured for absorption at 590 nm using an ELISA reader (made by Toyo Sokki K. K.).

As a result of measurement, it was found that 64 clones produced a specific antibody bound to UCB, not bound to the carrier protein. The clones thus selected above were then subjected to competitive saturation analysis to select clones showing a high reactivity to the UCB antigen. As a result, 16 clones were selected out of the 64 clones. The 16 clones were then measured for sub-classes in conventional manner and it was found that 7 clones belong to IgG1, 3 clones to IgG2a, 4 clones to IgG2b, 1 clone to IgG3, and 1 cone to IgGM.

The competitive saturation analysis was conducted for the purpose to determine a relative compatibility of the monoclonal antibody to various substances related to UCB. As a result, it has been found that the reactivity of all the monoclonal antibodies to UCB was inhibited to a remarkable extent by UCB-HSA in concentrations ranging from $10^{-8}$ to $10^{-6}$. More specifically, it has been found that the UCB which had been dissolved in the HSA solution only acted on only one clone, but little acted or did not act on all the other clones. Bilirubin monoglucuronate and bilirubin diglucuronate reacted with each of the monoclonal antibodies to a comparably good extent. Biliverdin, hemin, and dipyrroles did not show any inhibitive effect on the monoclonal antibodies but several ones which showed a weak reactivity. Cholic acid did not show any cross reactivity at concentrations up to $10^{-4}$M. Accordingly, the monoclonal antibodies according to the present invention has been found as a result of various analyses that they did not react with HSA coated on the plates and showed a specificity to the bilirubin and that HSA did not inhibit the reactivity of the UCB with the monoclonal antibodies. It has further been found that biliverdin and hemin little inhibited the reaction of the monoclonal antibodies regardless of a similarity in their chemical structures. Bile acid did not inhibit the reaction.

too.

Example 13: Preparation of Bilirubin Antibody:

BALB/c female mice were immuned by intraperitoneal injection with 100 μl (60 μg) of an emulsified mixture prepared by mixing the bilirubin-bound BSA obtained in Example 4 with the same amount of Freund's complete adjuvant. The spleen cells thus obtained from the mice were then treated in substantially the same manner as in Example 12, thus leading to the formation of hybridoma cells. The hybridoma cells in turn were treated in substantially the same manner as in Example 12 to give the desired monoclonal antibody.

Example 15: Preparation of Bilirubin Antibody:

BALB/c mice were immuned by intraperitonal injection with 100 μl (60 μg) of an emulsified mixture prepared by emulsifying the bilirubin-bound BSA obtained in Example 1 with the same amount of Freund's adjuvant. The mice were given additional immunization by injection at the daily rate of 200μl, 400μl, 400μl and 400 μl from four days prior to cell fusion. The spleen cells of the mice immuned above were then subjected to cell fusion with mice myeloma cells (P3-X63-Ag-U1) by means of the polyethylene glycol method. The fused cells were selected by incubation in the HAT medium and the resulting hybridoma cells producing the monoclonal antibody specific to the bilirubin were treated in conventional manner to yield the desired monoclonal antibody.

Example 16: Preparation of Bilirubin Antibody:

The procedures of Example 12 were followed in substantially the same manner with the exception that the bilirubin antigen was used other than that obtained in Example 11, thus yielding the desired monoclonal antibody.

Industrial Applicability

The use of the monoclonal antibody according to the present invention for bioassay provides various advantages. The monoclonal antibody according to the present invention has a high specificity to

bilirubin; it does not detect any other homolgue and dipyrrol derivatives; and it does not indicate at all such a non-specific reaction as shown in conventional diazo coupling method. Its sensitivity is as extremely high as 5 x $10^{-12}$ moles and higher than any other method by two orders. In these respects, the present invention provides the extremely useful advantage. Furthermore, this method offers the advantage that it does not require extraction as required in the HPLC method. The monoclonal antibody according to the present invention does not show any cross reactivity with bile acid, thus providing the possibility that it can be used for measuring bilirubins in the bile, too. It is also noted that the use of two kinds of the monoclonal antibodies according thereto permits a concurrent measurement for both total bilirubin and conjugated bilirubins. It is further to be noted that the use of the monoclonal antibodies presents the extremely useful advantage from the clinical standpoint that it permits a measurement for non-bound bilirubins, too, with high sensitivity and specificity. The monoclonal antibody, when bound to a stationary phase, can be used for a specific removal of the bilirubins from the serum of a life-risking disease or hyper-bilirubinemia, like the enzyme method using an immobilized bilirubin oxydase.

It is furthermore to be noted that the monoclonal antibody according to the present invention has a high specificity to each of particular bilirubins so that it permits a direct quantitation analysis of such bilirubins within an extremely short period of time and with high efficiency, such a direct analysis could not be made by any conventional methods. The use of the monoclonal antibody can detect the bilirubin without transformation or decomposition of its structure in vivo unlike any conventional method, thus permitting an accurate diagnosis of the cause and disease conditions of jaundice. This enables a diagnosis of latent metabolic abnormality or malformation immediately after delivery or even in the mother's womb.

They also may allow a diagnosis of blood diseases, liver diseases or malignant tumors so that they are extremely useful in terms of clinical diagnosis to such an extent that no other conventional diagnosis can compete.

# C L A I M S

1. A bilirubin antigen in which a bilirubin is bound to a protein through a spacer.

2. A bilirubin antigen as claimed in claim 1, wherein said bilirubin is bound at the carbon atom in the most external vinyl group on the pyrroline ring side of said bilirubin to the protein through the spacer.

3. A bilirubin antigen as claimed in claim 1, wherein said bilirubin is bound at at least one carboxyl group site to the protein.

4. A bilirubin antigen as claimed in claim 2, wherein the bilirubin antigen is represented by general formula:

bilirubin residue - $CH(CH_3)$-SP-N-PT

(in which "bilirubin residu" is a residue of the bilirubin from which vinyl group on the most external ring of the bilirubin is excluded; SP is a residue of the spacer; and PT is a residue of the protein).

5. A bilirubin antigen as claimed in claim 3, wherein the bilirubin antigen is represented by general formula:

bilirubin group - CO - NH - PT

or general formula:

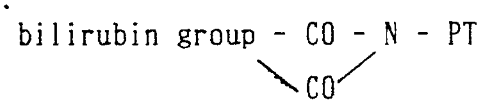

(in which "birirubin group" is a residue of the bilirubin from which one or two carboxyl group thereof is or are excluded; and PT is a residue of the protein).

6. A monoclonal antibody capable of recognizing a bilirubin bound to a protein with or without the aid of a spacer.

7. A process for preparing a monoclonal antibody characterized by producing from a hybridoma cell obtainable by fusion of an antibody-producing cell obtained by immunization with a bilirubin with a tumor cell capable of promulgating by division in a culture system.

8. Use of a monoclonal antibody characterized by using the

monoclonal antibody for recognizing a bilirubin antigen corresponding to the monoclonal antibody, said monoclonal antibody capable of recognizing a bilirubin bound to a protein with or without the aid of a spacer.

# INTERNATIONAL SEARCH REPORT

International Application No PCT/JP87/00999

**I. CLASSIFICATION OF SUBJECT MATTER** (if several classification symbols apply, indicate all) [3]

According to International Patent Classification (IPC) or to both National Classification and IPC

Int.Cl$^4$ C07K15/12, 15/04, A61K39/395, C12P21/00, G01N33/577, 33/72

**II. FIELDS SEARCHED**

Minimum Documentation Searched [4]

| Classification System | Classification Symbols |
|---|---|
| IPC | C07K15/00, A61K39/395, C12P21/00, G01N33/577, 33/72 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| X | J. Clin. Invest., Vol. 71, No. 6, (1983). Wolfgang Stremmel and five others, [Physicochemical and immnohistological studies of a sulfobromophthalein-and bilirubin-binding protein from rat liver plasma membranes], pp.1796-1805 | 1-5 |
| Y | JP, A, 61-5025 (Kukita Takeshi) 10 January 1986 (10. 01. 86) Pages 2 to 4 (Family: none) | 6-8 |

* Special categories of cited documents: [15]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance: the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search [2] | Date of Mailing of this International Search Report [2] |
|---|---|
| March 3, 1988 (03. 03. 88) | March 14, 1988 (14. 03. 88) |
| International Searching Authority [1] | Signature of Authorized Officer [20] |
| Japanese Patent Office | |